# EUROPEAN PATENT SPECIFICATION

(11) **EP 3 476 388 B1**
(45) Date of publication and mention of the grant of the patent: **29.09.2021**
(21) Application number: 18201332.6
(22) Date of filing: 18.10.2018
(51) Int. Cl.: A61K 31/122, A61K 9/16, A23L 33/10, A23L 29/00

(54) **COMPOSITION COMPRISING COENZYME Q10 AND PIPERINE**
ZUSAMMENSETZUNG UMFASSEND COENZYM Q10 UND PIPERIN
COMPOSITION COMPRENANT LA COENZYME Q10 ET LA PIPÉRINE

(30) Priority: 24.10.2017 IT 201700120582; 13.03.2018 IT 201800003503
(43) Date of publication of application: 01.05.2019
(73) Proprietor: Difass International S.p.A., 47853 Coriano (RN) (IT)
(72) Inventor: MARINI, Daniele, 47853 CORIANO (RN) (IT); GIORGINI, Davide, 47039 SAVIGNANO SUL RUBICONE (FC) (IT); AGOSTINI, Alida, 47853 CORIANO (RN) (IT)
(74) Representative: Bianchetti & Minoja SRL

(56) References cited:
- EP-A1- 3 165 218
- WO-A1-2014/016238
- US-A- 5 536 506
- US-A- 6 045 826
- US-A1- 2011 195 058
- DATABASE WPI Week 201754 Thomson Scientific, London, GB; AN 2017-431319 XP002779467, & CN 106 727 441 A (XIAMEN KINGDOMWAY BIOTECHNOLOGY CO LTD) 31 May 2017 (2017-05-31)
- VLADIMIR BADMAEV ET AL: "Piperine derived from black pepper increases the plasma levels of coenzyme q10 following oral supplementation", THE JOURNAL OF NUTRITIONAL BIOCHEMISTRY, vol. 11, no. 2, 1 February 2000 (2000-02-01), pages 109-113, XP055461836, AMSTERDAM, NL ISSN: 0955-2863, DOI: 10.1016/S0955-2863(99)00074-1
- SHAILENDRA WADHWA ET AL: "Bioavailability Enhancement by Piperine: A Review", ASIAN JOURNAL OF BIOMEDICAL AND PHARMACEUTICAL SCIENCES, vol. 04, no. 2014, 15 October 2014 (2014-10-15), pages 1-8, XP55461844, DOI: 10.15272/ajbps.v4i36.576

## Description

The invention relates to a composition for oral use in granular or microgranular form comprising coenzyme Q10 associated with piperine obtained from plant extracts and maltodextrins.

### PRIOR ART

Coenzyme Q10 (CoQ10) belongs to a family of substances known as coenzymes Q, which are present in the majority of aerobic organisms. Precisely because of their ubiquitous presence, in organisms ranging from bacteria to plants and the higher animals, said substances are also called ubiquinones. Chemically, ubiquinones are compounds characterised by a benzoquinone ring in the structure, which are differentiated from one another by the presence of an isoprenoid side chain; in humans, the chain comprises 10 isoprene units, thus defining coenzyme Q10.

CoQ10 or ubidecarenone (*trans* 2,3-dimethoxy-5-methyl-6-decaprenyl-1,4-benzoquinone) is synthesised by the body, and is therefore not considered to be an actual vitamin, although it is sometimes improperly called vitamin Q. CoQ10 plays a very important bioenergetic role in the cells, since it acts as cofactor at mitochondrial level in the electron transport chain for production of ATP. CoQ10 also possesses antioxidant activities against free radicals, which helps to preserve the integrity of the cell and mitochondrial membranes by protecting them against lipid peroxidation.

In view of said health properties, CoQ10 is commonly present on the market, especially in diet supplements (with a maximum daily limit of 200 mg) and cosmetic products. Its widespread use is also justified by the important role it performs at mitochondrial and bioenergetic level, and by its safety, confirmed by various clinical trials.

CoQ10 has been found to have various benefits, mainly in the cardiovascular and neurodegenerative fields. It is now known that HMG-CoA reductase-inhibiting medicaments (statins), the main class of molecules used in hyperlipidaemia, significantly lower the endogenous levels of CoQ10; this effect has been observed with various statins (*Banach et al., 2015*). This depletion can be a factor responsible for the myopathies correlated with statin use, and can give rise to problems, especially in patients with heart failure. Numerous investigations have demonstrated a close correlation between CoQ10 deficiency and severity of heart failure. A number of clinical trials and meta-analyses support the use of CoQ10 in heart failure, and the results of a recent, large-scale clinical trial confirmed an improvement in the symptoms, with a significant reduction in the main adverse effects and mortality (*Q-SYMBIO trial, 2014*). CoQ10 supplementation in these patients is therefore deemed appropriate and healthy.

The usefulness of CoQ10 in the treatment of hypertension is confirmed by numerous researchers.

CoQ10 is absorbed by the small intestine, and then conveyed by the lymphatic vessels, whence they enter the systemic circulation. The absorption of CoQ10 is highly variable; it is lower on an empty stomach and higher on a full stomach, especially when the diet has a high lipid content. However, the absorption efficiency of CoQ10 is generally low, because of its insolubility in water and its relatively high molecular weight. The compound is distributed in various body tissues, and can cross the blood-brain barrier. CoQ10 is mainly eliminated by the biliary and faecal routes (*Bhagavan et al., 2006*).

Piperine is the main pungent substance present in many plants of the *Piperaceae* family, other members of which include black pepper (*Piper nigrum L*.) and long pepper (*Piper longum L*.). Piperine is believed to possess anti-inflammatory effects. Piperine can increase the bioavailability of some nutritional substances and some medicaments in both animals and humans. It has been observed in some studies that single doses of piperine ranging from 20 and 50 mg, administered orally in concomitance with some medicaments, significantly increase the serum levels of those medicaments by reducing their clearance. Piperine has been observed to increase the levels and prolong the serum half-life of some nutritional substances such as CoQ10 (*Badmaev et al., 2000*) and beta carotene. The mechanism that gives rise to said effects is not yet fully understood. At higher doses it seems to be associated with the ability of piperine to inhibit the enzymes responsible for drug metabolism; at lower doses, however, this effect seems to be independent of enzyme inhibition, and it is postulated that in such cases piperine may act as a "thermonutrient", increasing the absorption of certain nutritional substances in the gastrointestinal tract by means of a local thermogenic action.

Turmeric (*C. longa* and *C. zodearia*) has traditionally been used in east Asian countries for its anti-inflammatory, wound-healing, digestive, antitumoral and antiviral properties *(*Araújo, Mem Inst Oswaldo Cruz 2001; 96: 723-8). Various pharmacological studies have also demonstrated the antiallergic and liver-protecting properties of extracts of the plant (Ram A, et al. Biol Pharm Bull 2003; 26: 1021-4*;* Matsuda H, et al. Bioorg Med Chem Lett 1998; 8: 339-44). In most cases, said pharmacological effects are attributable to curcumin, the main compound extracted from turmeric (Aggarwal BB, et al. Adv Exp Med Biol 2007; 595: 1-75).

Curcumin is known to exhibit low solubility in water and low intestinal absorption; it is also subject to high metabolism in the liver. These factors are responsible for the limited bioavailability of curcumin, which emerges after oral administration, even in amounts of up to 8-12 g. Some researchers have observed that concomitant administration of curcumin with piperine led to higher plasma concentrations of curcumin than the administration of curcumin alone, in both humans and animals (Shoba G et al. Planta Med. 1998 May; 64(4):353-6).

The use of phospholipid-curcumin complexes to obtain better absorption of curcumin is also known.

EP 2 228 062 A1 describes a composition comprising a phospholipid-curcumin and piperine complex for the treatment of drug resistance. The curcumin-phospholipid complex, due to its hydrophobic nature, is preferably used in products of a mainly hydrophobic nature, and can be difficult to apply in supplements in aqueous liquid form, or those for which water is used as the reconstitution medium. Its use is therefore limited to specific pharmaceutical forms.

Red yeast rice is the product of fermentation of rice by the fungus *Monascus purpureus,* which produces various substances that modulate the lipid level in the blood, known as "monacolins". The monacolin most active for these purposes is "monacolin K", the red yeast rice content of which depends on the strain of *Monascus purpureus* used and the fermentation conditions.

Many substances widely used for nutritional purposes are lipophilic (hydrophobic) or exhibit a high degree of solubility in oils and in many organic solvents, but are practically insoluble in water. The insolubility in water of bioactive lipophilic compounds can represent a major limitation on their nutritional applications by making effective administration extremely difficult.

A number of approaches can be taken to overcome these limitations. Many lipophilic substances are soluble in organic solvents, some of which are miscible in water, but many of said solvents are unfortunately incompatible with dietary use. Other organic solvents such as ethanol or propylene glycol are allowed for dietary use, but their use is restricted for food safety and compliance reasons, thus limiting their possible therapeutic applications.

Another approach is to incorporate the lipophilic substances in multiphase emulsions, mainly consisting of drops of hydrophobic solvents (wherein the hydrophobic active ingredient is dissolved), dispersed in a hydrophilic solvent (water), and surfactants, typically hydrophilic ones, that facilitate the wettability of hydrophobic powders, which would otherwise float if placed in water (e.g. talc or coenzyme Q10). The critical factor in the preparation of said compositions is to ensure not only correct homogenisation of the dispersed phase in the dispersing phase, but also long-term preservation of said systems, which are unstable by nature. Moreover, many of said surfactants are additives whose use, though allowed in the dietary field, can cause immune responses in some individuals.

These approaches have little value, and are complex, since administration by swallowing is required, and the use of aqueous solutions is the only acceptable way of ensuring an effective therapeutic approach.

In particular, approaches are known which improve the solubility and/or bioavailability of CoQ10, including the formation of complexes compatible with water.

For example, WO 9617626 relates to the composition of ubiquinones with polyoxyethanyl-cholesteryl sebacate (PCS) as solubilising agent, using as solvent tetrahydrofuran, which is subsequently evaporated and reconstituted in a suitable buffer solution. Unlike said composition, none of the preparations of the present invention require the use of organic solvents, thus eliminating successive evaporation stages and residues, which are potentially risky in long-term use, and also requiring fewer manufacturing stages, which reduces manufacturing costs.
Document EP 3 165 218 A1 discloses a CoQ10 composition where maltodextrin is used as an excipient, and which provides improved water-solubility of CoQ10.
US 6056971 relates to the increased dissolution and bioavailability of coenzyme Q10 achieved by using non-ionic solubilising agents and edible polyacrylic alcohol to provide a liquid composition in the form of gelatin capsules. Unfortunately, the use of said invention is restricted to a few pharmaceutical forms, namely those which are in liquid form or can incorporate liquid forms.
US 4483873 relates to an aqueous pharmaceutical solution comprising coenzyme Q10, soya lecithin and optionally propylene glycol as water-miscible solvent. Soya lecithin is widely used in the nutritional field as an emulsifier, as it is useful to promote the dispersion of hydrophobic compounds in water; however, allergic reactions can be triggered in some individuals due to its soya origin, the presence of which must be made clear to the consumer. Moreover, the use of water-miscible organic solvents, which is often frequent in pharmaceutical preparations, is subject to more restrictive limits in the nutritional field.
US 6045826 relates to compositions of lipophilic compounds rendered watersoluble, comprising a lipophilic compound, including coenzyme Q10, and a solubilising agent having both hydrophobic and lipophilic characteristics. In this case too, coenzyme Q10 is associated with pharmaceutically but not nutritionally acceptable carriers or diluents. Moreover, the multiple preparation stages of the compositions cited, some of which are repeated 2 or 3 times, involve significantly higher costs than the simple granulation with water described in the present invention.

Capsules and tablets are very common pharmaceutical forms in diet supplements, because they have the advantage of contributing nutritional substances in small portions with a few daily administrations.

Pharmaceutical forms requiring water as administration carrier are also fairly common in diet supplements. Pre-prepared powders, enclosed in sachets or in the caps of twin-chamber bottles, designed to be reconstituted shortly before administration, are an example of preparations that need water for correct administration. The use of pre-prepared sachets is necessary when the administration of large amounts of active ingredients (grams) is required, which would entail low compliance if they were taken in tablet or capsule form. Products in sachets designed to be reconstituted in water are particularly useful for individuals who, for medical or other reasons, have swallowing difficulties. In fact, in the case of powders designed to be reconstituted in water, the addition of suitable amounts of thickeners provides an easy way of modulating the consistency of the reconstituted solution. The paediatric target is particularly liable to swallowing difficulty.

Twin-chamber bottles are very useful in cases where it is desirable for the ingredients to be administered simultaneously in both liquid and solid form, when the characteristics of the latter prevent them from being included directly in aqueous solutions. One limitation of this format is the small space in the measuring cap, which prevents the inclusion of large amounts of powder. A particular advantage of the composition of the present invention is that it provides significant amounts of CoQ10 in the form of small amounts of granules compatible with aqueous solutions, which are therefore compatible with twin-chamber bottles.

A composition containing CoQ10 and piperine could therefore be advantageously used for health purposes and to improve the bioavailability of CoQ10 by boosting its activities, and therefore to promote the maintenance of the state of health. More advantageously, said composition would be desirable in an aqueous medium for ease of administration; even more desirable would be a composition that is stable and homogeneous in water, without substantial precipitation in the period prior to its consumption.

The invention relates to a composition and a kit comprising CoQ10 and piperine as disclosed in the appended claims.

### DESCRIPTION OF THE INVENTION

It has now been found that a combination of coenzyme Q10 (CoQ10) or a derivative thereof, in particular ubiquinol, and piperine can be advantageously formulated in granular or microgranular form using dietary maltodextrins only, thereby conveniently minimising the excipient content to ensure maximum safety of nutritional use.

The present invention provides a simple method of administering nutritionally significant amounts of CoQ10, either "as is" or using water or aqueous solutions as an administration medium, as it possesses better water-dispersibility characteristics than CoQ10 alone.

Said composition in granular or microgranular form also represents a rapid, low-cost solution, as it is made by a single, simple granulation process, using a conventional fluid-bed granulator.

The object of the invention is therefore a composition for oral use in water-dispersible granular or microgranular form, comprising CoQ10 and/or ubiquinol associated with piperine, optionally curcumin and maltodextrins, the latter being the only excipient.

According to a preferred aspect thereof, the invention relates to said composition in kit form, comprising:
- a liquid phase, for example in a bottle, and
- a powdered phase in the measuring cap of said bottle, said cap containing the water-dispersible granulate or microgranulate of CoQ10 associated with piperine and maltodextrins and curcumin.

According to a further aspect the formulations of the invention are presented, in sachets or stick packs designed to be reconstituted or administered directly, effervescent granulates or tablets, swallowable tablets, chewable tablets, hard capsules, soft capsules, film edibles, chewing gums or non-effervescent granulates.

According to a further aspect, the invention relates to diet supplements or medical foods or medical devices comprising said composition.

### BRIEF DESCRIPTION OF FIGURES

**Figure I** shows the turbidity of aqueous suspensions of the composition as described in Example 2 (CoQ10-pip) and coenzyme Q10 (CoQ10) only.
**Figure II** shows the turbidity kinetics of aqueous suspensions of the composition described in Example 2 (CoQ10-pip) and coenzyme Q10 (CoQ10) only.
**Figure III** shows the mean particle size distribution (n=5) in the aqueous suspension of the composition according to the invention as described in Example 2 (continuous curve), and the mean particle size distribution (n=5) of the same suspension filtered at 0.22 µm (dashed curve).
**Figure IV** shows the UV/visible absorption spectra of the composition according to the invention described in Example 2 (CoQ10-pip) "as is", fraction suspended after centrifugation, fraction suspended after filtration, and absorption spectrum of coenzyme Q10 (CoQ10) only, "as is" (top right).
**Figure V** shows the antioxidant action of the "conventional" coenzyme Q10 (CoQ10) and of the composition according to the patent (CoQ10-pip) as described in Example 2, represented as the value of hydrophilic ORAC (A) and lipophilic ORAC (B), expressed in µmol TE/g ± standard deviation (SD). (*) indicates that the two formulations have significantly different antioxidant activities (p≤0.05).

### DETAILED DESCRIPTION OF THE INVENTION

The compositions according to the invention consist of coenzyme Q10 and/or ubiquinol in a weight percentage from 10% to 20 %, piperine in a weight percentage from 0.02% to 0.5% , optionally curcumin in a weight percentage from 2% to 50% in granular or microgranular form.

The percentage weight content of CoQ10 and/or ubiquinol ranges from 10% to 20%, most preferably from 1.5% to 5%.

The percentage weight content of piperine ranges from 0.02 to 0.50%, preferably from 0.04% to 0.4%, most preferably from 0.06% to 0.21%. The percentage weight content of curcumin ranges from 2 to 50%, more preferably from 4% to 40%, most preferably from 6% to 21%.

Piperine can be present in pure form or in the form of an extract of a plant that contains it, in particular *Piper nigrum L.* or *Piper longum L.* extracts with a piperine weight content of up to 98%.

The curcumin can be in pure form or in the form of an extract of a plant that contains it, in particular *Curcuma longa* or *Curcuma zodearia* extracts wherein the curcumin weight content is up to 98%.

Piperine can be present in pure form or in the form of an extract of a plant that contains it, in particular *Piper nigrum L.* or *Piper longum L.* extracts with a piperine weight content of up to 95%.

The compositions according to the invention do not contain any excipients other than maltodextrins.

The granules according to the invention can be prepared in a fluid-bed granulator by granulating the mixture of CoQ10, piperine and optionally curcumin and a percentage of maltodextrins with a granulating solution consisting of water and the remaining percentage of maltodextrins.

The compositions of the invention have a good degree of dispersion in water, greater than that of CoQ10 alone, without the use of further excipients, supported by experimental data.

The following examples illustrate the invention in more detail.

### Example 1. Preparation of granulate

A mixture of powders containing coenzyme Q10 and piperine, together with a percentage of maltodextrins as diluent, was prepared.

The mixture is prepared, accurately weighing the powders, in a pre-heated rack of a fluid-bed granulator, and heated until an optimum temperature of 40-45°C is reached. The remaining part of the maltodextrins is dissolved in water to form the granulating solution, which is sprayed onto the mixture in the granulator through nozzles. At the end of the drying and cooling process, a granulate with the qualitative/quantitative composition set out in Table I is obtained:

**Table I**

| | |
|---|---|
| Coenzyme Q10 | 20% |
| *Piper nigrum* extract with 95% piperine content | 0.87% |
| Maltodextrin | 79.13% |

### Example 2. Preparation of granulate with low piperine content

As piperine is a substance with poor organoleptic compliance, due to its typical pungent flavour, perceptible even in small amounts when administered orally, a second, more advantageous composition, characterised by a low percentage of piperine in water-dispersible granular or microgranular form, was devised to further improve the flavour of the composition according to the invention. This latter composition is reported in Table II:

**Table II**

| | |
|---|---|
| Coenzyme Q10 | 20% |
| *Piper nigrum* extract with 95% piperine content | 0.44% |
| Maltodextrin | 79.56% |

### Example 3. Comparative determination of water-dispersibility

The water-dispersibility characteristics were analysed and quantified by turbidimetric measurement and determination of particle-size distribution by DLS (Dynamic Light Scattering), analysis methods in accordance with the reports in the literature (*Matsushita et al., 2013*). Turbidimetric analysis establishes the turbidity level of a suspension by exploiting the absorption and reflection of the particles, while size distribution determines the size of its particles. These tests were conducted on the composition according to the invention by comparison with "conventional" dietary CoQ10, in the form of a crystalline powder with a high ubidecarenone content (>98%), present on the market and distributed by Fagron Italia Srl.

The comparative turbidimetric measurement was conducted by mixing in water the samples of the composition according to the invention and CoQ10 alone, stirring manually for a few seconds. The dispersions were centrifuged, and the suspended fractions were taken up and immediately analysed by turbidimetry. The results are shown in Figure I. Moreover, to delineate the turbidity kinetics of the suspensions, further measurements were subsequently acquired every 10 minutes between 20 and 60 minutes after the initial mixing, and finally after 75 minutes. The curves shown in Figure II were obtained. All the measurements were obtained with a BioPhotometer Plus spectrophotometer (Eppendorf) with a fixed wavelength.

The DLS determinations of the size distribution of the composition according to the invention and of CoQ10 alone in suspension were conducted with a Zetasizer Nano ZS instrument (Malvern), and the curves are shown in Figure III. The samples were prepared in water, stirring manually for a few seconds, then centrifuged and analysed by DLS. To characterise the suspension further, the suspended fraction was filtered through a nylon membrane with a porosity of 0.22 µm, and then analysed by DLS by the same method as described above. The size characterisation results are set out in Table III.

Further information in support of the different dispersibilities of the composition according to the invention compared with CoQ10 alone was provided by determination of the UV/visible absorption spectrum of the two samples, dispersed at 10 mg/mL, "as is", after centrifugation and after filtration. The absorption curves are shown in Figure IV.

**Table III.**

| Sample | Maximum diameter (mean ± standard deviation. nm) | Polydispersity index |
|---|---|---|
| CoQIO-pip | 394 ± 141 | 0.839 |
| CoQ10-pip (filtrate) | 19 ± 10 | 0.214 |
| CoQ10 | n.d. | n.d. |

### Example 4. Comparative evaluation of antioxidant activity by ORAC test

To evaluate how water dispersion influences the antioxidant activity of coenzyme Q10, an ORAC test was effected to compare the antioxidant activity of CoQ10 alone (CoQ10) with that of the composition of the invention as described in Example 2 (CoQ10-pip). The ORAC values are expressed as micromoles of Trolox equivalents per gram of sample (µmol TE/g). The ORAC test was conducted according to the protocol for hydrophilic substances and for lipophilic substances. The test results are set out in Figure V.

### Example 5. Preparation of granulate comprising curcumin

A mixture of powders was prepared containing coenzyme Q10, piperine and curcumin together with a percentage of maltodextrins as diluent.
The mixture is prepared, accurately weighing the powders, in a pre-heated rack of a fluid-bed granulator, and heated until an optimum temperature of 40-45°C is reached. The remaining part of the maltodextrins is dissolved in water to form the granulating solution, which is sprayed onto the mixture in the granulator through nozzles. At the end of the drying and cooling process, a granulate with the qualitative/quantitative composition set out in Table IV is obtained:

**Table IV**

| | |
|---|---|
| Coenzyme Q10 | 5 mg |
| Piperine from *Piper nigrum* extract with 95% piperine content | 0.20 mg |
| Curcumin from *Curcuma longa* extract with 95% curcuminoid content | 20 mg |
| Maltodextrin | q.s. to 100 mg |

### Example 6. Comparative determination of water-dispersibility of the granulate of example 5

The water-dispersibility characteristics were analysed and quantified by turbidimetric measurement and determination of particle-size distribution by DLS (Dynamic Light Scattering), analysis methods in accordance with the reports in the literature (*Matsushita et al., 2013*). Turbidimetric analysis establishes the turbidity level of a suspension by exploiting the absorption and reflection of the particles, while size distribution determines the size of its particles. These tests were conducted on the composition according to the invention by comparison with "conventional" dietary CoQ10, in the form of a crystalline powder with a high ubidecarenone content (>98%), present on the market and distributed by Fagron Italia Srl, and with dried extract of *Curcuma longa* L., 95% content, distributed by Labiotre.

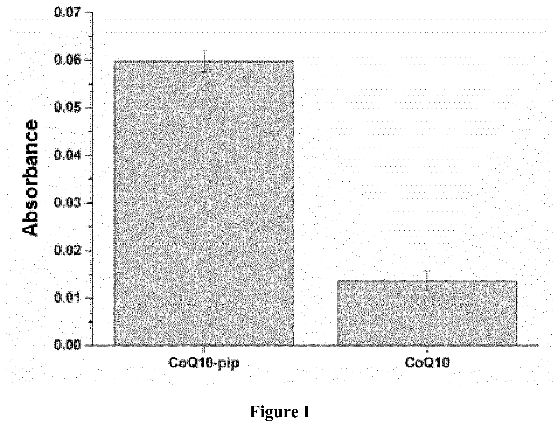

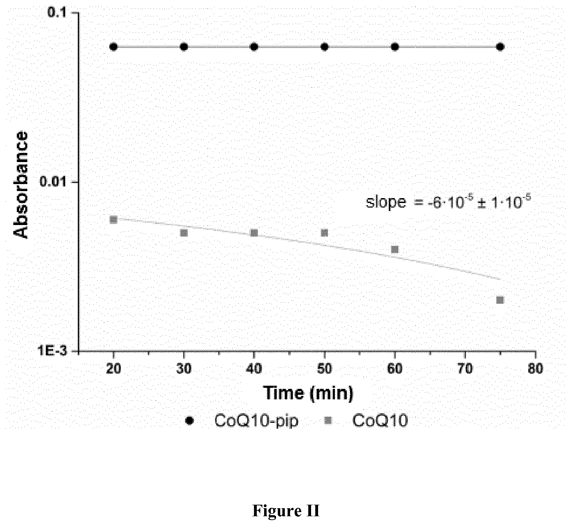

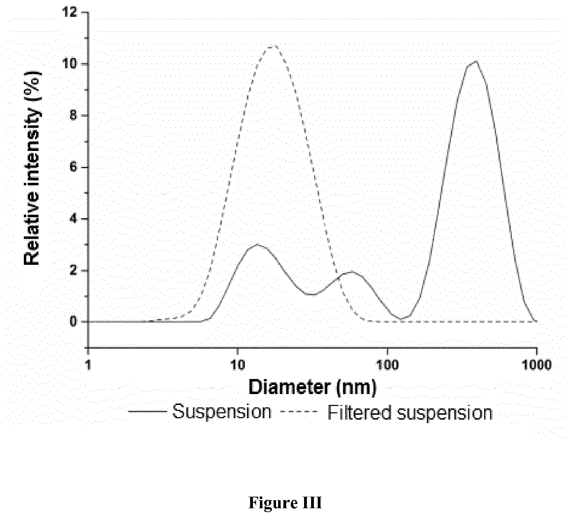

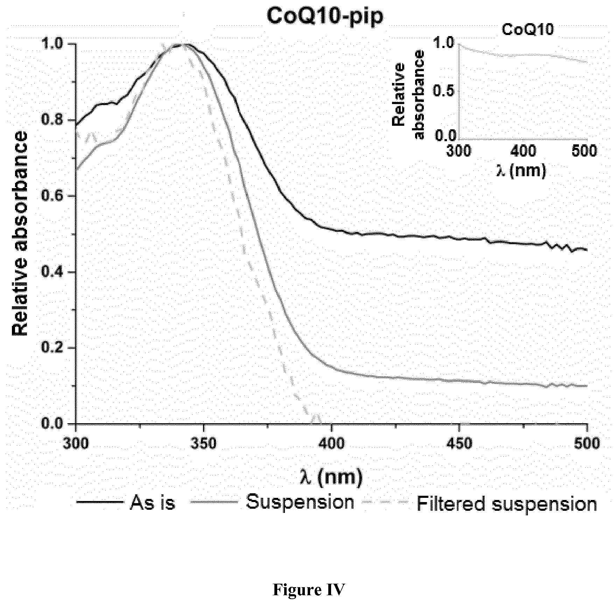

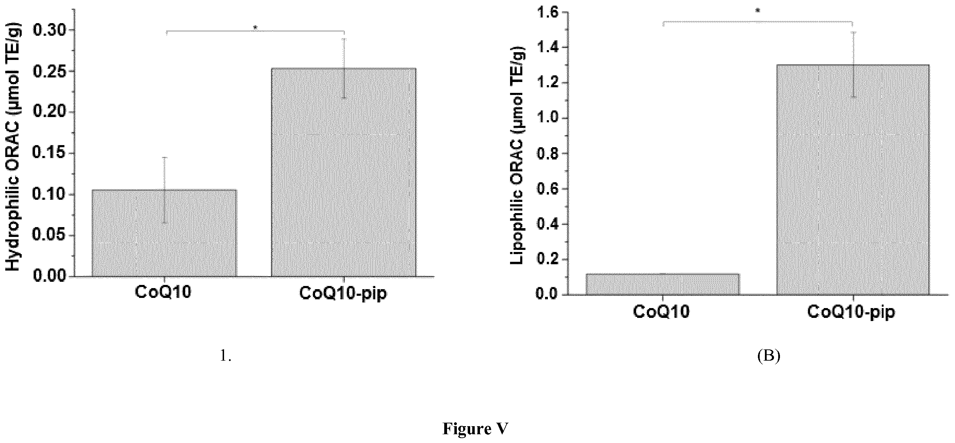

## Claims

1. Composition for oral use in granular or microgranular form consisting of coenzyme Q10 and/or ubiquinol in a weight percentage from 10% to 20 %, piperine in a weight percentage from 0.02% to 0.5% , optionally curcumin in a weight percentage from 2% to 50% and maltodextrin as a suitable nutritionally acceptable excipient, in the absence of other excipients.

2. Composition according to claim 1, wherein said granulate or microgranulate is formulated in sachets or stick packs either for reconstitution or for direct administration, swallowable tablets, chewable tablets, hard capsules, soft capsules, effervescent tablets or granulates, edible films, chewing gums or non-effervescent granulates.

3. A kit comprising:
- a nutritionally acceptable liquid phase and
- a powdered solid phase in a measuring cap, said cap containing part or all of a composition according to any one of claims 1-2.

## Patentansprüche

1. Zusammensetzung zur oralen Anwendung in granularer oder mikrogranularer Form bestehend aus Coenzym Q10 und/oder Ubiquinol in einem Gewichtsprozentanteil von 10 % bis 20 %, Piperin in einem Gewichtsprozentsatz von 0,02 % bis 0,5 %, gegebenenfalls Curcumin in einem Gewichtsprozentsatz von 2 % bis 50 % und Maltodextrin als geeignetes ernährungsphysiologisch akzeptabler Hilfsstoff, in Abwesenheit von anderen Hilfsstoffen.

2. Zusammensetzung nach Anspruch 1, wobei das Granulat oder Mikrogranulat in Beuteln oder Stickpackungen entweder zur Rekonstitution oder zur direkten Verabreichung, schluckbaren Tabletten, Kautabletten, Hartkapseln, Weichkapseln, Brausetabletten oder -granulaten, essbaren Filmen, Kaugummis oder nicht-brausenden Granulaten enthalten ist.

3. Ein Bausatz bestehend aus:
- einer ernährungsphysiologisch akzeptablen flüssigen Phase und
- einer pulverförmigen festen Phase in einer Meßkappe, wobei die Kappe einen Teil oder die gesamte Zusammensetzung nach einem der Ansprüche 1-2 enthält.

## Revendications

1. Composition à usage oral sous forme granulaire ou microgranulaire constituée de coenzyme Q10 et/ou d'ubiquinol en un pourcentage en poids de 10 % à 20 %, de pipérine en un pourcentage en poids de 0,02 % à 0,5 %, éventuellement de curcumine en un pourcentage en poids de 2 % à 50 % et de maltodextrine comme excipient approprié acceptable sur le plan nutritionnel, en l'absence d'autres excipients.

2. Composition selon la revendication 1, dans laquelle ledit granulé ou microgranulé est formulé en sachets ou en sachets tubulaires soit pour reconstitution soit pour administration directe, comprimés à avaler, comprimés à croquer, gélules, capsules molles, comprimés ou granulés effervescents, films comestibles, gommes à mâcher ou granulés non effervescents.

3. Kit comprenant :
- une phase liquide acceptable sur le plan nutritionnel et
- une phase solide en poudre dans un bouchon doseur, ledit bouchon contenant tout ou partie d'une composition selon l'une quelconque des revendications 1 à 2.
